# EUROPÄISCHE PATENTSCHRIFT

(11) **EP 0 728 454 B1**
(45) Veröffentlichungstag und Bekanntmachung des Hinweises auf die Patenterteilung: **08.12.1999**
(21) Anmeldenummer: 96101489.1
(22) Anmeldetag: 02.02.1996
(51) Int. Cl.: A61F 13/00, A61F 13/08

(54) **Verfahren zum Befestigen von Polstern an medizinischen Bandagen**
Method for fixing cushioning pads to medical bandages
Méthode pour fixer des coussinets de rembourrage à des bandages médicaux

(30) Priorität: 22.02.1995 DE 19506128
(43) Veröffentlichungstag der Anmeldung: 28.08.1996
(73) Patentinhaber: Beiersdorf Aktiengesellschaft, 20245 Hamburg (DE)
(72) Erfinder: Bodenschatz, Stefan, Dr., D-21614 Buxtehude (DE)

(56) Entgegenhaltungen:
- EP-A- 0 262 638
- EP-A- 0 496 071
- DE-A- 2 342 149
- DE-C- 3 832 438
- GB-A- 874 360

## Beschreibung

Die Erfindung betrifft ein Verfahren zum Befestigen von Polstern oder Pelotten an medizinischen Bandagen, z.B. für orthopädische Zwecke, Kompressionsstrümpfen, Bandagen, welche nach Verbrennungen getragen werden, und dgl.. Derartige Produkte bestehen überlicherweise aus elastischen Materialien, vorzugsweise Gewirken oder Gestricken, und die Polster bzw. Pelotten können je nach Verwendungszweck als Druckpolster, bei Bewegung auch massierend wirkende Druckpolster oder schützende und stützende Polster ausgebildet sein. Sie bestehen meist aus elastischen Materialien, wie Silikonkautschuk oder Schaumgummi, oder aus polsternden Materialien, wie Vlies, und können die verschiedensten Formen aufweisen.

Diese Polster werden üblicherweise innen oder außen an den Bandagen befestigt, indem sie mit einem Überzug abgedeckt werden, der etwa die Kontur des Polsters aufweist und an seinem Umfangsrand an der Bandage angenäht wird. Gemäß der DE-C 38 32 438 ist es auch bekannt, den Überzug in der Weise zu Befestigen, daß dieser auf seiner dem Polster zugewandten Seite mit einer elastischen, thermoplastischen Kunststoffbeschichtung versehen ist und durch Erhitzen im Bereich der Ränder mit dem Textilmaterial der Bandagen verklebt wird. Das Verschweißen bzw. Verkleben von verschiedenen Textilmaterialien durch thermoplastische Beschichtungen oder Zwischenschichten, auch thermoplastisch elastischen, ist dabei eine in der Testiltechnik bereits seit langem bekannte Arbeitsweise, wie z.B. aus der DE-OS 14 60 008 und DE-OS 23 42 149 u.a..

Bei den bekannten Methoden zur Befestigung von Polstern müssen diese und der lose Überzug genau positioniert und befestigt werden. Dies ist um so schwieriger, je unregelmäßiger die Kontur des Polsters ist, wie dies bei anatomischgerecht gestalteten Polstern oft der Fall ist, und je kleiner das Polster oder die Pelotte ist und je unzugänglicher ihre Position am Produkt ist.

Aufgabe der Erfindung war es, die Befestigung von Polstern oder Pelotten so zu gestalten, daß dies einfach und auch an schwierig zugänglichen Stellen der Bandage gut möglich ist. Unter Polstern oder Pelotten sollen dabei jede Art von Formkörpern verstanden werden, welche auf Gelenke, Muskeln oder Sehnen einwirken sollen. Meist bestehen sie aus Silikonkautschuk, Synthesekautschuk, Gummi, Schaumstoff oder anderen mehr oder weniger elastischen Materialien, z.B. auch Vliesstoffen. Aber auch Druckpelotten mit einem integrierten kleinen sog. Friktionskern aus einem harten oder inkompressiblen Material gemäß der EP-PS 496 071 bzw. US.P. 5 306 229 oder nur ein derartiger mehr oder weniger harter Kern, welcher die verschiedensten Ausgestaltungen aufweisen kann und bei Bewegung eine besonders wirksame und örtlich gezielte Massage ausübt, fallen darunter. Der Einfachheit halber soll im folgenden in der Regel generalisierend nur von Polstern gesprochen werden aber alle diese möglichen Ausführungsformen gemeint sein.

Gelöst wird diese Aufgabe durch das Verfahren gemäß Anspruch 1.

Hierbei wird das Polster in einem ersten Arbeitsgang in eine der Kontur desselben entsprechende Tasche eingepackt.

Zumindest auf der später dem Produkt zugewandten Seite des Polsters wird ein thermoplastisches, vorzugsweise thermoplastisch-elastisches Trägermaterial in Form von Folie, Gewebe, Gewirke oder Vlies eingesetzt. Derartige Materialien sind bekannt und bestehen z.B. aus Polyurethanen, Polyvinylchlorid oder Polyethylen.

Auf der anderen Seite wird bevorzugt ein elastisches hautfreundliches Material, z.B. eine Folie, Maschenware oder ein Gewebe aufgelegt. Dies insbesondere dann, wenn das Polster auf der Innenseite der Bandage angebracht wird und damit auf die Haut zu liegen kommt.

Entsprechend der Kontur des Polsters werden die beiden Materialien durch lokales Erhitzen und damit Erweichen des thermoplastischen Materials miteinander verbunden. Das Erhitzen kann beispielsweise nach dem Heißsiegelverfahren mit erhitzten Stempeln oder vorzugsweise mit dem Hochfrequenzverfahren erfolgen.

Im gleichen oder in einem nachfolgenden Arbeitsgang wird das eingehüllte Polster entsprechend seiner Kontur ausgeformt, d.h. vorzugsweise ausgestanzt oder durch ein anderes Trennverfahren herausgetrennt. Dabei ist darauf zu achten, daß die Schweiß- bzw. Siegelnaht nicht verletzt wird. Vorzugsweise wird ein Rand von etwa 2 - 20 mm, vorteilhaft 4 - 7 mm, überstehen gelassen. Im Falle der beschriebenen Friktionsskerne bzw. kleinen Formkörper zur gezielten Massage kann die Größe der Tasche auch nur ca. 1 cm betragen und der überstehende Rand nur ca. 1 mm betragen.

In einem weiteren Arbeitsgang werden die auf die angegebene Weise ummantelten Polster mittels der thermoplastischen Seite, vorzugsweise nur über den an der Außenkontur überstehenden Rand, an der Bandage befestigt. Dies geschieht erneut durch Wärmeeinwirkung. Dabei kann eine Verbindung im gesamten Bereich, in dem das Polster an der Bandage anliegt, erfolgen oder nur entlang des überstehenden Randes. Auch eine nur partielle Befestigung in der Fläche oder vor allem im Randbereich kann je nach anatomischer Ausformung der Bandage, z.B. für den Schulterbereich, günstig sein. Hierdurch kann eine gewisse Beweglichkeit des Polsters erreicht werden. Dabei kann der Randbereich an den Stellen, welche zur Befestigung dienen, etwas vergrößert sein, um eine besonders stabile Verbindung zu ermöglichen.

Im Falle der kleinen Formkörper zur gezielten Massage erfolgt aufgrund der geringen Größe der Tasche eine Verbindung vorzugsweise im gesamten Bereich, in dem das Polster an der Bandage anliegt.

Das später der Bandage zugewandte, das Polster umhüllende Trägermaterial kann mit einer oder mehreren Aussparungen beliebiger Form, z.B. rund oder oval, oder auch Unterbrechungen wie z.B. Schlitzen versehen sein. Dadurch wird bei Bandagen, die im Gebrauch gedehnt werden, der Einfluß dieses thermoplastischen und vorzugsweise elastischen Trägermaterials auf die Eigenschatten des Produkts nach dem Befestigen des Polsters gezielt steuerbar, z.B. durch eine erhöhte Dehnbarkeit und Nachgiebigkeit. Die Öffnung oder Öffnungen dürfen jedoch nur so groß gestaltet werden, daß das Polster vor und während der Befestigung an der Bandage noch sicher in der Tasche gehalten wird.

Werden zum Umhüllen des Polsters, auch Pelotte genannt, dichte Materialien, wie Folien oder beschichtete Gewebe und Gewirke, eingesetzt, so kann anstelle einer festen Pelotte auch eine Flüssigkeit, z.B. in Form eines Gels oder hochviskosen Öls, gemäß dem beschriebenen Verfahren eingepackt und befestigt werden. Dies ergibt Pelotten, die eine sehr gleichmäßige Druckverteilung ermöglichen. Eine weitere Möglichkeit besteht darin, die entstandene Kammer mit Luft zu füllen.

Die erfindungsgemäß eingeschweißten bzw. eingesiegelten oder eingeklebten Polster können direkt weiterverarbeitet, d.h. an den jeweiligen Bandagen oder dgl. befestigt werden, sie können aber auch zwischengelagert und erst bei entsprechendem Bedarf verarbeitet werden. Dies ist besonders dann wirtschaftlich vorteilhaft, wenn die Endfertigung der Bandagen räumlich getrennt von der Herstellung und Ausrüstung der Polster stattfindet.

Bei besonders einfach gestalteten Bandagen und Polstern können die beschriebenen Arbeitsgänge auch gleichzeitig durchgeführt werden. D.h., auf die Bandage wird die thermoplastische Schicht gelegt, darauf das Polster, darüber das bereits weitgehend formgerechte Abdeckmaterial und dann wird in einem Schweißvorgang entlang des überstehenden Randes das Abdeckmaterial mittels der thermoplastischen Schicht an der Bandage befestigt.

Die Zeichnungen zeigen die einzelnen Herstellungs- und Befestigungsschritte der Polster beispielhaft.

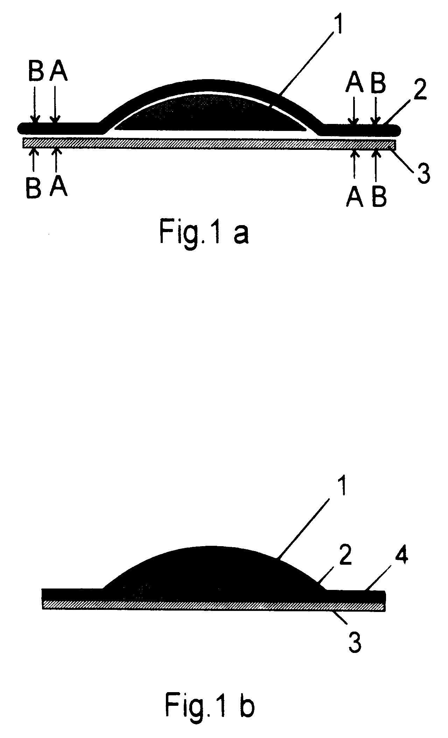

In der Figur 1 a wird das Einschweißen eines Polsters dargestellt. Dabei bedeuten (1) das Polster, (2) das Deckmaterial, (3) das thermoplastische Trägermaterial, (A) die Schweißstelle, an welcher die Hochfrequenzelektroden angesetzt werden, (B) die Stanzstelle. Figur 1 b zeigt das eingeschweißte Polster mit dem überstehenden Rand (4).

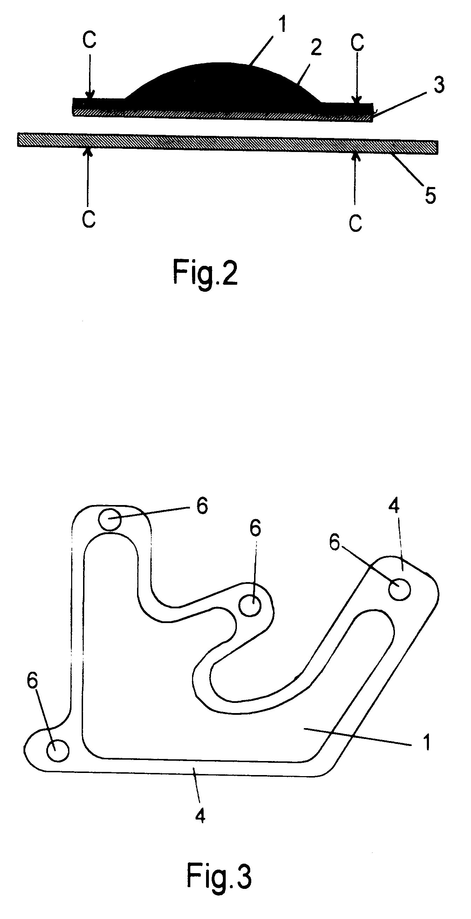

Figur 2 zeigt den Befestigungsvorgang an der Bandage. Dabei bedeuten wieder (1) das Polster, (2) das Deckmaterial und (3) das thermoplastische Trägermaterial. Mit (5) ist der Bandagenstoff bezeichnet und mit (C) die Schweißstelle, an welcher das Polster an der Bandage befestigt wird.

Figur 3 zeigt ein kompliziert gestaltetes Polster (1), welches mit seinem überstehenden und z.T. verbreiterten Rand (4) an einzelnen Befestigungspunkten (6) an einer nicht dargestellten Bandage befestigt ist.

Das erfindungsgemäße Verfahren ermöglicht es, Polster und Pelotten an Bandagen jeglicher Form auf eine technisch einfache und wirtschaftlich sparsame Art und Weise zu befestigen, wobei auch komplizierte Ausgestaltungen keine größeren Probleme darstellen.

## Patentansprüche

1. Verfahren zum Befestigen von Polstern an medizinischen Bandagen, Strümpfen und dgl., dadurch gekennzeichnet, daß das Polster (1) zwischen zwei Trägermaterialien (2 und 3) aus Folie, Gewebe, Gewirke oder Vlies, von welchen mindestens eines aus einem thermoplastischen Material besteht, durch Wärmeeinwirkung eingeschweißt bzw. -geklebt wird, im wesentlichen entsprechend seiner Kontur ausgeformt wird und durch Wärmeeinwirkung mittels des thermoplastischen Materials (3), welches der Bandagenseite zugewandt ist, an der Bandage (5) befestigt wird.

2. Verfahren gemäß Anspruch 1, dadurch gekennzeichnet, daß das zwischen den beiden Trägermaterialien (2 und 3) eingeschweißte Polster (1) entsprechend seiner Kontur mit einem überstehenden Rand (4) ausgestanzt, ausgeschnitten oder dgl. wird.

3. Verfahren gemäß Anspruch 1 und 2, dadurch gekennzeichnet, daß das Polster (1) mittels des an der Außenkontur überstehenden Randes (4) an der Bandage (5) befestigt wird.

4. Verfahren gemäß Anspruch 3, dadurch gekennzeichnet, daß das Polster (1) nur an einzelnen Stellen (6) des Randbereiches (4) an der Bandage (5) befestigt wird.

5. Verfahren gemäß Anspruch 4, dadurch gekennzeichnet, daß der Randbereich (4) an den Stellen, an welchen das Polster (1) befestigt ist, vergrößert ist.

6. Verfahren gemäß einem oder mehreren der vorangehenden Ansprüche, dadurch gekennzeichnet, daß die thermoplastische Folie, das Gewebe, Gewirke oder Vlies aus einem elastischen, thermoplastischen Material besteht.

7. Verfahren gemäß einem oder mehreren der vorangehenden Ansprüche, dadurch gekennzeichnet, daß das thermoplastische Trägermaterial, welches der Bandage zugewandt ist, mit einer oder mehreren Aussparungen oder Unterbrechungen versehen ist.

8. Verfahren gemäß einem oder mehreren der vorangehenden Ansprüche, dadurch gekennzeichnet, daß das Polster in Form eines harten kleinen Formkörpers ausgebildet ist.

9. Verfahren gemäß einem oder mehreren der vorangehenden Ansprüche, dadurch gekennzeichnet, daß die Trägermaterialien (2 und 3), zwischen denen das Polster (1) eingeschweißt bzw. -geklebt ist, dicht gegenüber dem Material ist, aus welchem das Polster besteht.

10. Vorrichtung für Bandagen und dgl., dadurch gekennzeichnet, daß sie aus einem Polster besteht, das zwischen zwei Trägermaterialien (2 und 3) eingeschweißt bzw. -geklebt ist gemäß einem oder mehreren der vorangehenden Ansprüche.

## Claims

1. Method for fixing cushioning pads to medical bandages, hoses and the like, characterized in that the cushioning pad (1) is welded or bonded by the action of heat between two support materials (2 and 3) made of foil, woven fabric, knitted fabric or nonwoven fabric, of which at least one consists of a thermoplastic material, is shaped essentially according to its contour and is fixed by the action of heat to the bandage (5) by means of the thermoplastic material (3) which faces the bandage side.

2. Method according to Claim 1, characterized in that the cushioning pad (1) welded between the two support materials (2 and 3) is punched out, cut out, or similar, according to its contour with a protruding margin (4).

3. Method according to Claims 1 and 2, characterized in that the cushioning pad (1) is fixed to the bandage (5) by means of the margin (4) protruding on the external contour.

4. Method according to Claim 3, characterized in that the cushioning pad (1) is fixed to the bandage (5) only at individual locations (6) on the margin area (4).

5. Method according to Claim 4, characterized in that the margin area (4) is enlarged at the locations where the cushioning pad (1) is fixed.

6. Method according to one or more of the preceding claims, characterized in that the thermoplastic foil, the woven fabric, the knitted fabric or nonwoven fabric consists of an elastic, thermoplastic material.

7. Method according to one or more of the preceding claims, characterized in that the thermoplastic support material which faces the bandage is provided with one or more cutouts or breaks.

8. Method according to one or more of the preceding claims, characterized in that the cushioning pad is designed in the form of a small, hard shaped article.

9. Method according to one or more of the preceding claims, characterized in that the support materials (2 and 3), between which the cushioning pad (1) is welded or bonded, are sealed with respect to the material from which the cushioning pad is made.

10. Device for bandages and the like, characterized in that it consists of a cushioning pad which is welded or bonded between two support materials (2 and 3) according to one or more of the preceding claims.

## Revendications

1. Méthode pour fixer des coussinets de rembourrage à des bandages médicaux, des manchons et similaires, caractérisée en ce que le coussinet de rembourrage (1) est soudé ou collé en position sous l'effet de la chaleur entre deux matériaux de support (2 et 3) sous forme de film, de tissu, de tissu à malles ou de nappe, dont au moins un se compose de matériau thermoplastique, est mis en forme essentiellement en fonction de son contour et est fixé au bandage (5) sous l'effet de la chaleur au moyen du matériau thermoplastique (3), qui est tourné du côté du bandage.

2. Méthode selon la revendication 1, caractérisée en ce que le coussinet de rembourrage (1) soudé entre les deux matériaux de support (2 et 3) est découpé à l'emporte-pièce, coupé ou similaire, en fonction de son contour, avec un bord dépassant (4).

3. Méthode selon la revendication 1 ou 2, caractérisée en ce que le coussinet de rembourrage (1) est fixé au bandage (5) au moyen du bord dépassant (4) sur le contour extérieur.

4. Méthode selon la revendication 3, caractérisée en ce que le coussinet de rembourrage (1) n'est fixé au bandage (5) qu'en des positions individuelles (6) de la zone périphérique (4).

5. Méthode selon la revendication 4, caractérisée en ce que la zone du bord (4) est agrandie aux endroits où le coussinet de rembourrage (1) est fixé.

6. Méthode selon l'une ou plusieurs des revendications précédentes, caractérisée en ce que le film, le tissu, le tissu à mailles ou la nappe thermoplastique se compose d'un matériau thermoplastique élastique.

7. Méthode selon l'une ou plusieurs des revendications précédentes, caractérisée en ce que le matériau de support thermoplastique, qui est tourné vers le bandage, est pourvu d'un ou de plusieurs évidements ou interruptions.

8. Méthode selon l'une ou plusieurs des revendications précédentes, caractérisée en ce que le coussinet de rembourrage est conçu en forme de petit corps de moulage dur.

9. Méthode selon l'une ou plusieurs des revendications précédentes, caractérisée en ce que les matériaux de support (2 et 3) entre lesquels le coussinet de rembourrage (1) est soudé ou collé sont serrés contre le matériau constituant le coussinet de rembourrage.

10. Dispositif pour bandages et similaires, caractérisé en ce qu'il se compose d'un coussinet de rembourrage qui est soudé ou collé entre deux matériaux de support (2 et 3) selon l'une ou plusieurs des revendications précédentes.
